# EUROPEAN PATENT APPLICATION

(11) **EP 4 740 864 A1**
(43) Date of publication of application: **13.05.2026**
(21) Application number: 24211547.5
(22) Date of filing: 07.11.2024
(51) Int. Cl.: A61B 6/00, G16H 30/40

(54) **PROVIDING GUIDANCE FOR ACQUIIRING A MEDICAL IMAGE**

(71) Applicant: Koninklijke Philips N.V., 5656 AG Eindhoven (NL)
(72) Inventor: BYSTROV, Daniel, Eindhoven (NL); KROENKE-HILLE, Sven, Eindhoven (NL); MENSER, Bernd, 5656AG Eindhoven (NL); VON BERG, Jens, Eindhoven (NL); SENEGAS, Julien Thomas, Eindhoven (NL)
(74) Representative: Philips Intellectual Property & Standards

(57) **Abstract**

A computer-implemented method of providing guidance for acquiring a medical image, is provided. The method includes: receiving camera image data representing an anatomical region (120) within a field of view of a medical imaging system; analysing the camera image data to determine a presence, or an absence, of an anatomical orientation marker (150) within the field of view of the medical imaging system; and outputting guidance for acquiring a medical image of the anatomical region (120) using the medical imaging system. The guidance is generated based on the presence, or the absence, of the anatomical orientation marker (150).

## Description

### FIELD OF THE INVENTION

The present disclosure relates to providing guidance for acquiring a medical image. A computer-implemented method, a computer program product, and a system, are disclosed.

### BACKGROUND OF THE INVENTION

Prior to medical image acquisition, anatomical orientation markers are typically placed within the field of view of the medical imaging system in order to indicate the orientation of an anatomical region that will be captured in the subsequently-acquired medical image.

For instance, prior to X-ray image acquisition, so-called anatomical side markers "ASMs", also known as X-ray lead markers, or simply as lead markers, are typically placed on the X-ray detector, or on the patient table, beside an anatomical region. The markers are placed by a radiographer, and they indicate the orientation of the anatomical region that will be captured in the subsequently-acquired X-ray image. The markers may indicate the anatomical side of a subject (e.g. left versus right). The markers may indicate the pose of the anatomical region relative to the X-ray imaging system (e.g. anterior - posterior "AP" versus posterior - anterior "PA"). The markers are formed from a radiopaque material such as lead, and consequently provide distinct features in the X-ray image. The markers provide confirmation of the anatomical origin of the X-ray image to a reviewing radiologist that reviews the image.

However, it can happen that a medical practitioner accidently mis-places, or simply forgets to place, an anatomical orientation marker. For instance, in some cases, a radiographer may accidentally mix-up a left anatomical side marker with a right anatomical side marker. In other cases, the radiographer may simply forget to place an anatomical side marker. This presents challenges during the subsequent workflow. In some situations, a mistake might be noticed by the radiographer whilst the subject is still in the imaging room. In such situations, the mistake can be rectified by including the correct marker, or by replacing the marker with the correct marker, and then acquiring a further X-ray image. This, however, exposes the subject to unnecessary additional X-ray dose. In other situations, a mistake might only be noticed after the subject has left the imaging room. In such situations, the mistake can confuse the radiologist that reviews the X-ray image, resulting in the need to recall the subject to the imaging room in order to acquire a further X-ray image. This, likewise, exposes the subject to unnecessary additional X-ray dose and also creates an unnecessary delay in workflow.

Consequently, there is a need for improvements in the acquisition of medical images when anatomical orientation markers are used.

### SUMMARY OF THE INVENTION

According to one aspect of the present disclosure, a computer-implemented method of providing guidance for acquiring a medical image, is provided. The method includes:
- receiving camera image data representing an anatomical region within a field of view of a medical imaging system;
- analysing the camera image data to determine a presence, or an absence, of an anatomical orientation marker within the field of view of the medical imaging system; and
- outputting guidance for acquiring a medical image of the anatomical region using the medical imaging system, wherein the guidance is generated based on the presence, or the absence, of the anatomical orientation marker.

In the above method, camera image data is analysed in order to determine a presence, or an absence, of an anatomical orientation marker within the field of view of the medical imaging system. Guidance is then based on the presence, or the absence, of the anatomical orientation marker. The guidance may relate to the presence, or to the absence, or to the suitability of a position, or to a recommended position, or to a suitability of a type, of the anatomical orientation marker, for example. The guidance helps to avoid mistakes relating to the placement of anatomical orientation markers. Since the guidance is generated by analysing camera image data, the guidance may be provided prior to the acquisition of a medical image, and thereby avoid mistakes in a subsequently-acquired medical image that might otherwise confuse a physician that reviews the medical image, or result in the need to recall the subject to the imaging room in order to acquire a further medical image.

In some embodiments, the method comprises receiving marker requirement information representing one or more requirements of the anatomical orientation marker. The guidance is generated based further on the one or more requirements of the anatomical orientation marker. Examples of such requirements include: a need to include the anatomical orientation marker in the medical image; an expected position of the anatomical orientation marker with respect to the anatomical region; and an expected type of the anatomical orientation marker. Generating the guidance based on the one or more requirements of the anatomical orientation marker enables the guidance to be tailored to a specific anatomical orientation marker.

In some embodiments, the guidance is generated based on the presence of the anatomical orientation marker, and the outputting guidance comprises one or more of: outputting an indication of a presence of the anatomical orientation marker; outputting an indication of a suitability of a position of the anatomical orientation marker with respect to the anatomical region; outputting an indication of a recommended position of the anatomical orientation marker with respect to the anatomical region; and outputting an indication of a suitability of a type of the anatomical orientation marker for the anatomical region. Outputting such indications helps to avoid mistakes in a subsequently-acquired medical image that might otherwise confuse a physician that reviews the medical image, or result in the need to recall the subject to the imaging room in order to acquire a further medical image.

In some embodiments, the analysing of the camera image data comprises determining, from the camera image data, a type and/or a current orientation of the anatomical region within the field of view of the medical imaging system. The guidance is generated based on the type and/or the current orientation of the anatomical region. Generating such guidance helps to avoid mistakes relating to the type and/or the current orientation of the anatomical region captured in a subsequently-acquired medical image. This may help to avoid the need to recall the subject to the imaging room in order to acquire a further medical image.

In some embodiments, the analysing of the camera image data comprises determining a current orientation of the anatomical region within the field of view of the medical imaging system. The outputting of the guidance comprises outputting, based on a comparison between the current orientation of the anatomical region and a reference orientation of the anatomical region, an indication of a suitability of the current orientation and/ or an indication of one or more adjustments to the current orientation for obtaining the reference orientation. Generating such guidance helps to avoid mistakes relating to the current orientation of the anatomical region captured in a subsequently-acquired medical image. This may help to avoid the need to recall the subject to the imaging room in order to acquire a further medical image.

In some embodiments, the method further comprises receiving orientation requirement information representing the reference orientation of the anatomical region. The orientation requirement information enables the guidance to be tailored to a specific reference orientation.

In some embodiments, the guidance is generated based on the absence of the anatomical orientation marker. The outputting of the guidance comprises one or more of: outputting an indication of an absence of the anatomical orientation marker; outputting an indication of a recommendation to include an anatomical orientation marker in the medical image; outputting an indication of a recommendation not to include an anatomical orientation marker in the medical image; outputting an indication of a recommended position of an anatomical orientation marker in the medical image; and outputting a prompt requesting user confirmation to include a virtual anatomical orientation marker in a proposed position in the medical image. Outputting such indications helps to avoid mistakes in a subsequently-acquired medical image that might otherwise confuse a physician that reviews the medical image, or result in the need to recall the subject to the imaging room in order to acquire a further medical image.

In some embodiments, the analysing of the camera image data comprises detecting the anatomical orientation marker and/or an orientation of the anatomical region, using an object detection algorithm. An object detection algorithm may be used to reliably detect the anatomical orientation marker and/or the orientation of the anatomical region.

In some embodiments, the analysing of the camera image data comprises inputting the camera image data into a machine learning algorithm and generating the guidance using the machine learning algorithm in response to the inputting. The machine learning algorithm is trained to generate the guidance using training data comprising a plurality of instances of camera image data, each instance of camera image data representing an anatomical region within a field of view of a medical imaging system; and wherein the training data further comprises for each instance of camera image data, corresponding guidance for acquiring a medical image of the anatomical region using the medical imaging system, the corresponding guidance being determined based on the presence, or the absence, of an anatomical orientation marker within the field of view of the medical imaging system. The machine learning algorithm provides a reliable way of generating the guidance. Moreover, its training data may be readily acquired from historic imaging procedures. This enables the machine learning algorithm to be trained in an efficient manner.

In some embodiments, the medical image is an X-ray image. Since the method provides guidance for acquiring an X-ray image of the anatomical region, the method may avoid mistakes that lead to an additional X-ray dose to a subject as a result of the need to recall the subject in order to acquire a further X-ray image.

In some embodiments, the camera image data is acquired by a camera having a field of view overlapping the field of view of the medical imaging system. The camera image data may be acquired using a (video) camera, such as a visible camera, a (near-) infrared camera, a depth camera, for example.

In some embodiments, the anatomical orientation marker is an X-ray anatomical side marker. An X-ray anatomical side marker is also known as an X-ray lead marker, or a lead marker, i.e. a marker formed from lead.

According to another aspect of the present disclosure, a computer program product is provided. The computer program product includes instructions which when executed by one or more processors, cause the one or more processors to carry out any embodiments of the method of providing guidance for acquiring a medical image. The advantages associated with the features of the method are provided correspondingly by the features of the computer program product.

According to another aspect of the present disclosure, a system for providing guidance for acquiring a medical image, is provided. The system comprises one or more processors configured to:
- receive camera image data representing an anatomical region within a field of view of a medical imaging system;
- analyse the camera image data to determine a presence, or an absence, of an anatomical orientation marker within the field of view of the medical imaging system; and
- output guidance for acquiring a medical image of the anatomical region using the medical imaging system, wherein the guidance is generated based on the presence, or the absence, of the anatomical orientation marker.

The advantages associated with the features of the method are provided correspondingly by the features of the system.

Further aspects, features, and advantages of the present disclosure will become apparent from the following description of examples, which is made with reference to the accompanying drawings.

### BRIEF DESCRIPTION OF THE DRAWINGS

Fig. 1 is a flowchart illustrating an example of a computer-implemented method of providing guidance for acquiring a medical image, in accordance with some aspects of the present disclosure.
Fig. 2 is a schematic diagram illustrating an example of a system 200 for providing guidance for acquiring a medical image, in accordance with some aspects of the present disclosure.
Fig. 3 is a schematic diagram illustrating an example of left X-ray anatomical side marker (left) and a right X-ray anatomical side marker (right), in accordance with some aspects of the present disclosure.
Fig. 4 is a schematic diagram illustrating an example of a camera image representing a foot within a field of view of the X-ray imaging system illustrated in Fig. 1, in accordance with some aspects of the present disclosure.
Fig. 5 is an example of an X-ray image 170 of a foot that has been acquired using the X-ray imaging system illustrated in Fig. 1, in accordance with some aspects of the present disclosure.

### DETAILED DESCRIPTION OF EMBODIMENTS

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer-implemented method, may be implemented in a computer program product, and in a system, in a corresponding manner.

In the following description, reference is made to examples of a computer-implemented method of providing guidance for acquiring a medical image. In some examples, reference is made to the provision of guidance for acquiring a medical image of a foot. The foot serves as an example of an anatomical region. It is, however, to be appreciated that that the method may be used to provide guidance for acquiring a medical image of any anatomical region. Thus, the method may also be used to provide guidance for acquiring a medical image of anatomical regions such as the chest, the arm, the leg, the knee, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed in the computer-implemented methods disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations performed in the computer-implemented methods disclosed herein.

As mentioned above, there is a need for improvements in the acquisition of medical images when anatomical orientation markers are used.

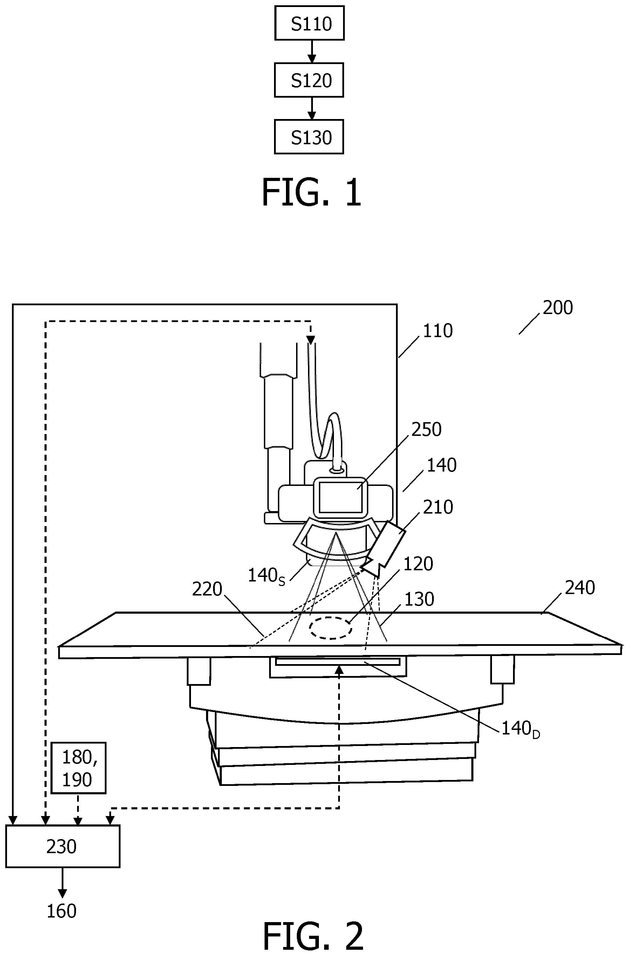

Fig. 1 is a flowchart illustrating an example of a computer-implemented method of providing guidance for acquiring a medical image, in accordance with some aspects of the present disclosure. Fig. 2 is a schematic diagram illustrating an example of a system 200 for providing guidance for acquiring a medical image, in accordance with some aspects of the present disclosure. It is noted that operations that are described in relation to the method illustrated in Fig. 1, may also be performed by the one or more processors 230 of the system 200 illustrated in Fig. 2. Likewise, operations that are described as being performed by the one or more processors 230 of the system 200 illustrated in Fig. 2, may also be performed in the method described with reference to Fig. 1. With reference to Fig. 1, and Fig. 2, the computer-implemented method of providing guidance for acquiring a medical image, includes:
- receiving S110 camera image data 110 representing an anatomical region 120 within a field of view 130 of a medical imaging system 140;
- analysing S120 the camera image data 110 to determine a presence, or an absence, of an anatomical orientation marker 150, 150' within the field of view 130 of the medical imaging system 140; and
- outputting S130 guidance 160 for acquiring a medical image 170 of the anatomical region 120 using the medical imaging system 140, wherein the guidance 160 is generated based on the presence, or the absence, of the anatomical orientation marker 150, 150'.

In the above method, camera image data is analysed in order to determine a presence, or an absence, of an anatomical orientation marker within the field of view of the medical imaging system. Guidance is then based on the presence, or the absence, of the anatomical orientation marker. The guidance may relate to the presence, or to the absence, or to the suitability of a position, or to a recommended position, or to a suitability of a type, of the anatomical orientation marker, for example. The guidance helps to avoid mistakes relating to the placement of anatomical orientation markers. Since the guidance is generated by analysing camera image data, the guidance may be provided prior to the acquisition of a medical image, and thereby avoid mistakes in a subsequently-acquired medical image that might otherwise confuse a physician that reviews the medical image, or result in the need to recall the subject to the imaging room in order to acquire a further medical image.

The operations that are performed in the method illustrated in Fig. 1, are described in more detail below.

Referring initially to the operation S110 illustrated in Fig. 1; in this operation, camera image data 110 is received. The camera image data 110 represents an anatomical region 120 within a field of view 130 of a medical imaging system 140.

In general, the camera image data 110 that is received in the operation S110 may represent a single camera image, or it may represent a (live) temporal sequence of camera images. The camera image data may be generated by various types of cameras. The camera image data may be generated by a (video) camera that is sensitive to one or more optical wavelength intervals within the visible and/or infrared portion of the optical spectrum, for example. The camera image data 110 may alternatively be generated by a different type of (video) camera, such as a hyperspectral camera, or a so-called depth camera. A depth camera generates camera image data that represents a range between the camera and objects within the camera's field of view. Various types of depth cameras are known, including depth cameras that employ a time-of-flight, or LIDAR principle, depth cameras that employ a structured light principle, and depth cameras that employ a binocular stereo vision principle.

With continued reference to the operation S110, in general, the anatomical region that is represented by the camera image data 110 may be any anatomical region. By way of some examples, the anatomical region may be a portion of a foot, or a portion of the chest, the arm, the leg, the knee, and so forth.

With continued reference to the operation S110, the medical imaging system 140 may be an X-ray imaging system. In general, an X-ray imaging system includes an X-ray source and a corresponding X-ray detector. The X-ray source and the X-ray detector of the X-ray imaging system may be provided in a variety of different configurations. For instance, in one example configuration the X-ray source is mounted to a ceiling via a gantry, and the corresponding X-ray detector is mountable to a patient bed. In this regard, the X-ray imaging system may be the DigitalDiagnost C90, or the Radiography 7300 C, X-ray imaging system marketed by Philips Healthcare, Best, The Netherlands, for example. In another example configuration, the X-ray source is mounted to a ceiling via a gantry, and the corresponding X-ray detector is mounted to a stand and held in a vertical position. In another example configuration, the X-ray imaging system may be a so-called mobile X-ray imaging system in which the X-ray source is mounted to a mobile unit, e.g. a cart, and the corresponding X-ray detector is able to adopt a remote position with respect to the X-ray source. The X-ray source and the corresponding X-ray detector may alternatively have a different configuration to these examples.

An example of the operation of receiving S110 camera image data 110 representing an anatomical region 120 within a field of view 130 of a medical imaging system 140, is illustrated in Fig. 2. In this example, the medical imaging system 140 has a field of view 130. The field of view 130 defines the extent of a volume from which the medical imaging system 140 is capable of acquiring medical image data. In the example illustrated in Fig. 2, the camera image data 110 is acquired by a camera 210 having a field of view 220 overlapping the field of view 130 of the medical imaging system 140. In the example illustrated in Fig. 2, the camera 210 is a video camera 220 that is sensitive to one or more optical wavelength intervals within the visible portion of the optical spectrum. The acquired camera image data 110 represents the anatomical region 120, which may be a portion of a foot, for example, within the field of view 130 of the medical imaging system 140.

In general, the spatial relationship between the field of view 220 of the camera, and the field of view 130 of the medical imaging system is known. For instance, the spatial relationship between the field of view 220 of the camera 210, and the field of view 130 of the medical imaging system, may be known by coupling the camera 210 to an element of the medical imaging system 140, such as the X-ray source 140s of the X-ray imaging system 140. In this case, the spatial relationship between the field of view 220 of the camera 210, and the field of view 130 of the medical imaging system, is fixed. Alternatively, the spatial relationship between the field of view 220 of the camera, and the field of view 130 of the medical imaging system, may be known by arranging that the field of view of the camera encompasses elements of the medical imaging system. For instance, the field of view 220 of the camera 210 may encompass a portion of the patient bed 240, or a portion of the X-ray source 140s, or a portion of the X-ray detector 140_{D}, of the X-ray imaging system 140. In this case, the camera 210 may be disposed elsewhere in the medical imaging room. For example, the camera 210 may be coupled to the wall, to a gantry, or to the ceiling, of the medical imaging room. In this case, the spatial relationship between the field of view 220 of the camera 210, and the field of view 130 of the medical imaging system, is derivable from the elements of the medical imaging system that are captured in the camera image data 110.

With continued reference to the operation S110 illustrated in Fig. 1; the camera image data 110 that is received in this operation may be received by one or more processors. The camera image data 110 may be received by the one or more processors 230 illustrated in Fig. 2 for example. In general, the camera image data 110 may be received via any form of data communication. For instance, the camera image data 110 may be received via wired, or wireless, or optical fiber communication. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

Referring now to the operation S120 illustrated in Fig. 1; in this operation, the camera image data 110 is analysed to determine a presence, or an absence, of an anatomical orientation marker 150, 150' within the field of view 130 of the medical imaging system 140.

In general, an anatomical orientation marker provides an indication of the orientation of an anatomical region that will be, or has been, captured in a subsequently-acquired medical image. Owing to mistakes that may arise with its placement, the anatomical orientation marker may be said to provide an indication of the intended orientation of an anatomical region that will be, or has been, captured in a subsequently-acquired medical image. The anatomical orientation marker may provide an indication of the anatomical side of the body (i.e. left versus right). Alternatively, or additionally, the anatomical orientation marker may provide an indication of the pose of the anatomical region relative to the imaging system (e.g. anterior - posterior "AP" versus posterior - anterior "PA"). If the subsequently-acquired medical image is an X-ray image, the anatomical orientation marker may be referred to as an X-ray anatomical side marker. An X-ray anatomical side marker "ASM", is also known as an X-ray lead marker, or simply as a lead marker. An X-ray anatomical side marker may provide an indication of the anatomical side of the body (i.e. left versus right) that will be captured in the subsequently-acquired medical image. An X-ray anatomical side marker may provide this indication via one or more letters (e.g. L versus R), or via alternative symbol(s). Alternatively, or additionally, an X-ray anatomical side marker may provide an indication of the pose of the anatomical region relative to the X-ray imaging system (e.g. anterior - posterior "AP" versus posterior - anterior "PA"). An X-ray anatomical side marker may provide this indication via one or more letters (e.g. AP versus PA), or via alternative symbol(s). X-ray anatomical side markers are formed from a radiopaque material such as lead, and consequently provide distinct features in the X-ray image. The markers provide confirmation of the anatomical origin of the X-ray image to a reviewing radiologist that reviews the image.

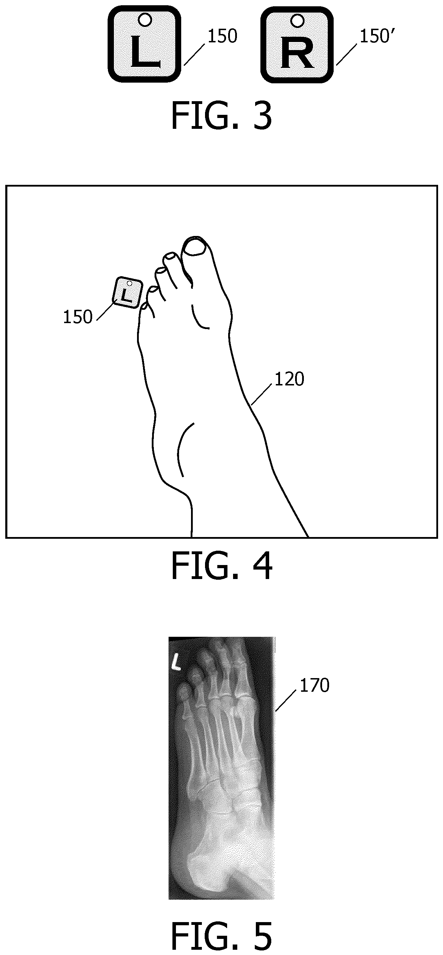

Some examples of X-ray anatomical side markers are illustrated in Fig. 3, which is a schematic diagram illustrating an example of left X-ray anatomical side marker (left) and a right X-ray anatomical side marker (right), in accordance with some aspects of the present disclosure. As illustrated in Fig 3, in these examples, the X-ray anatomical side markers provide an indication of indication of the anatomical side of the body (i.e. left versus right) that will be captured in the subsequently-acquired medical image, specifically via the corresponding letters L, and R. In this example, the letters are formed from a radiopaque material, e.g. lead, or stainless steel, that is embedded in a radio-translucent material, e.g. epoxy. Consequently, the letters appear in bright contrast in an X-ray image. It is noted that the examples illustrated in Fig. 3 are only provided by way of examples, and that X-ray anatomical side markers may alternatively provided in a different form. For instance, the letters may instead provide an indication of the pose of the anatomical region relative to the X-ray imaging system (e.g. anterior - posterior "AP" versus posterior - anterior "PA"), or the letters may be provided in the form of a cutout from a radiopaque material.

Thus, the presence, or the absence, of a variety of different types of anatomical orientation markers may be determined in the operation S120.

An example of the camera image data 110 that is analysed in the operation S120 is illustrated in Fig. 4, which is a schematic diagram illustrating an example of a camera image representing a foot within a field of view of the X-ray imaging system illustrated in Fig. 1, in accordance with some aspects of the present disclosure. In this example, the camera image data 110 includes a live temporal sequence of camera images representing the foot within the field of view 130 of the X-ray imaging system 210 illustrated in Fig. 2. In this example, a left X-ray anatomical side marker is correctly placed with respect to the left foot, and so the result of the analysing operation S120 may be to provide a confirmation that an anatomical side marker is present within the field of view 130 of the medical imaging system 140.

Various techniques may be used to analyse the camera image data in the operation S120. In general, these techniques include image processing techniques. For example, classical machine vision techniques, and also machine learning techniques may be used. Examples of these techniques are described below.

Referring now to the operation S130 illustrated in Fig. 1; in this operation, guidance 160 is outputted for acquiring a medical image 170 of the anatomical region 120 using the medical imaging system 140. The guidance 160 is generated based on the presence, or the absence, of the anatomical orientation marker 150, 150'.

Various types of guidance may be generated, and outputted in the operation S130. For instance, in some examples, the guidance 160 is generated based on the presence of the anatomical orientation marker 150, 150'. In these examples, the outputting S130 of the guidance may include one or more of:
- outputting an indication of a presence of the anatomical orientation marker 150, 150';
- outputting an indication of a suitability of a position of the anatomical orientation marker 150, 150' with respect to the anatomical region 120;
- outputting an indication of a recommended position of the anatomical orientation marker 150, 150' with respect to the anatomical region 120; and
- outputting an indication of a suitability of a type of the anatomical orientation marker 150, 150' for the anatomical region 120.

Outputting such indications helps to avoid mistakes in a subsequently-acquired medical image that might otherwise confuse a physician that reviews the medical image, or result in the need to recall the subject to the imaging room in order to acquire a further medical image.

In this example an indication of a presence of the anatomical orientation marker 150 may be generated depending on whether or not a marker has been detected in the camera image data. The indication may be provided as a straightforward binary (e.g. yes/ no) indication. For instance, a green icon may be displayed on a display device to confirm that an anatomical orientation marker has been detected, whereas a red icon may be displayed if no marker has been detected. In this example, an indication of a suitability of a position of the anatomical orientation marker 150, 150' with respect to the anatomical region 120 may be generated based on a comparison between a detected position of the marker in the camera image data, and a boundary defining a range of acceptable reference positions for the marker. If the marker is detected within the boundary, then an indication in the form of a green icon may be outputted, whereas if the marker is outside the boundary, a red icon may be outputted. In this example, an indication of a recommended position of the anatomical orientation marker 150, 150' with respect to the anatomical region 120 may be generated using anatomical region-specific template, and based on a determination of a type of the anatomical region that is detected in the camera image data. The anatomical region-specific template includes a boundary that defines a range of acceptable reference positions for the marker. The boundary may be outputted, e.g. in as an overlay on a graphical representation of the camera image data, in order to indicate the recommended position of the anatomical orientation marker.

With reference to the example illustrated in Fig. 3, and in which the left X-ray anatomical side marker is correctly placed with respect to the left foot; in this example, the guidance that is outputted may include one or more of: an indication that the anatomical orientation marker 150 is present; an indication that the position of the anatomical orientation marker 150 with respect to the anatomical region 120 is correct; an indication of the recommended position of the anatomical orientation marker 150 with respect to the anatomical region 120 (e.g. the recommended position may be indicated via a boundary that encompasses the current position of the marker); and an indication that the left anatomical orientation marker 150 is indeed suitable for use with the foot 120.

In other examples, the guidance 160 is generated based on the absence of the anatomical orientation marker 150, 150'. In these examples, the outputting S130 of the guidance may include one or more of:
- outputting an indication of an absence of the anatomical orientation marker 150, 150';
- outputting an indication of a recommendation to include an anatomical orientation marker 150, 150' in the medical image 170;
- outputting an indication of a recommendation not to include an anatomical orientation marker 150, 150' in the medical image 170;
- outputting an indication of a recommended position of an anatomical orientation marker 150, 150' in the medical image 170; and
- outputting a prompt requesting user confirmation to include a virtual anatomical orientation marker in a proposed position in the medical image.

Outputting such indications helps to avoid mistakes in a subsequently-acquired medical image that might otherwise confuse a physician that reviews the medical image, or result in the need to recall the subject to the imaging room in order to acquire a further medical image.

In this example, an indication of an absence of the anatomical orientation marker 150, 150' may be generated depending on whether or not a marker has been detected in the camera image data. In this example, an indication of a recommendation to include an anatomical orientation marker 150, 150' in the medical image 170 may be generated based on a determination of a type of the anatomical region that is detected in the camera image data. For instance, some anatomical regions may be deemed not to require an anatomical marker because the orientation of the anatomical region is undisputable in a subsequently-generated medical image. In this example, an indication of a recommendation not to include an anatomical orientation marker 150, 150' in the medical image 170, may similarly be generated based on a determination of a type of the anatomical region that is detected in the camera image data. In this example, an indication of a recommended position of an anatomical orientation marker 150, 150' in the medical image 170 may be generated using the anatomical region-specific template described above, and based on a determination of a type of the anatomical region that is detected in the camera image data. In this example, a prompt requesting user confirmation to include a virtual anatomical orientation marker in a proposed position in the medical image may be generated using the anatomical region-specific template described above, and based on a determination of a type of the anatomical region that is detected in the camera image data. In this case, the anatomical region-specific template includes a virtual anatomical orientation marker in a proposed location, rather than a boundary. In response to the user confirmation to include the virtual anatomical orientation marker in the proposed position, the marker may then be included in the medical image. This saves time because it avoids the need for a clinician, e.g. a radiographer, to physically place the marker within the field of view of the medical imaging system.

The guidance 160 may be outputted in various ways in the operation S130. In general, the guidance may be outputted in any human-intelligible form. For instance, the guidance 160 may be outputted in the form of a graphical representation, e.g. in the form of an icon, or as text. In this case, the guidance 160 may be outputted to a display device, such as a display that is coupled to the X-ray imaging source 140s, or a monitor, or a virtual! augmented reality display device, for example. The guidance 160 may alternatively be outputted in other ways, such as in the form of an audio alarm, or an audio message. In this case, the guidance 160 may be outputted to an audio device, such as a loudspeaker, earphones, and so forth. In so doing, the guidance helps to avoid mistakes relating to the placement of anatomical orientation markers that might otherwise confuse a physician that reviews a medical image, or that might result in the need to recall the subject to the imaging room in order to acquire a further medical image.

As mentioned above, in some examples, the medical imaging system 140 is an X-ray imaging system. In these examples, the corresponding guidance 160 that is outputted in the operation S130 is guidance for acquiring an X-ray image 170 of the anatomical region 120.

Since the method provides guidance for acquiring an X-ray image of the anatomical region, the method may avoid mistakes that lead to an additional X-ray dose to a subject as a result of the need to recall the subject in order to acquire a further X-ray image.

Subsequent to the outputting of the guidance in the operation S130, a physician may acquire a medical image 170 of the anatomical region 120 using the medical imaging system 140. By way of an example, Fig. 5 is an example of an X-ray image 170 of a foot that has been acquired using the X-ray imaging system illustrated in Fig. 1, in accordance with some aspects of the present disclosure. The arrangement of the foot 120 and the left X-ray anatomical side maker 150 correspond to their arrangement in the camera image data illustrated in Fig. 4. Thus, following the guidance that the anatomical side maker 150 is present, and is correctly positioned, and is suitable for use with the foot 120, the physician has acquired the X-ray image 170.

As mentioned above, various techniques may be used to analyse the camera image data in the operation S120. In general, these techniques include image processing techniques. Both classical machine vision techniques, and also machine learning techniques may be used.

In one example, the operation of analysing S120 the camera image data 110 comprises detecting the anatomical orientation marker 150, 150' and/or an orientation of the anatomical region 120, using an object detection algorithm.

An object detection algorithm may be used to reliably detect the anatomical orientation marker and/or the orientation of the anatomical region.

In this example, the object detection algorithm may employ various image processing techniques, including both classical machine vision techniques, and also machine learning techniques. For instance, in order to detect an anatomical orientation marker, objection detection algorithms that are based on convolutional neural network "CNN" architecture, or a You Only Look Once "YOLO" architecture, may be trained to using camera images that include various examples of anatomical orientation markers. By way of an example, the output of the object detection algorithm may be to provide an indication of the presence of an anatomical orientation marker and/or an indication of its position in a camera image. In order to detect an orientation of the anatomical region, various known pose estimation algorithms may be used, including AlphaPose, OpenPifPaf, and OpenPose, for example. By way of an example, the output of the object detection algorithm may be to provide an indication that a foot that is captured in the camera image data is a left foot, and that an outer side of the foot is facing the X-ray source 140s, for example. The object detection algorithm may provide this output in the form of a stick diagram representing the bones in the foot, for example. As described in the examples below, this information may be used to provide guidance, such as an indication of the presence, or absence, of an anatomical orientation marker, a suitability of a position of the anatomical orientation marker with respect to an anatomical region, and so forth.

In another example, the operation of analysing S120 the camera image data 110 comprises inputting the camera image data 110 into a machine learning algorithm and generating the guidance 160 using the machine learning algorithm in response to the inputting. In this example, the machine learning algorithm is trained to generate the guidance 160 using training data comprising a plurality of instances of camera image data, each instance of camera image data representing an anatomical region within a field of view of a medical imaging system; and wherein the training data further comprises for each instance of camera image data, corresponding guidance for acquiring a medical image of the anatomical region using the medical imaging system, the corresponding guidance being determined based on the presence, or the absence, of an anatomical orientation marker within the field of view of the medical imaging system.

The machine learning algorithm provides a reliable way of generating the guidance. Moreover, its training data may be readily acquired from historic imaging procedures. This enables the machine learning algorithm to be trained in an efficient manner.

The training data in this example may be acquired from historic medical imaging procedures using the arrangement illustrated in Fig. 2, for example. For instance, camera image data may be acquired using the camera 210 during multiple imaging procedures. The corresponding guidance may be acquired from records indicating whether or not, or why, an imaging procedure was repeated. Synthetic examples of correctly-placed, and also mis-placed anatomical orientation markers, incorrect anatomical orientation markers, absences of anatomical orientation markers, and so forth may be generated by having an operator of the medical imaging system (e.g. a radiographer) simulate such mistakes. The corresponding synthetic guidance may be provided by the operator. The guidance may correspond to the examples described above, e.g. the guidance may include an indication of a presence of the anatomical orientation marker, an indication of an absence of the anatomical orientation marker, and so forth.

Various types of machine learning algorithms may be used in accordance with this example. For instance, the machine learning algorithm may be provided by a neural network that includes a convolutional neural network "CNN" architecture, a recurrent neural network "RNN" architecture, a variational autoencoder "VAE" architecture, a transformer architecture, or a combination thereof.

In general, the training of a neural network involves inputting training data (in the above example, the instances of camera image data, into the neural network), and iteratively adjusting the neural network's parameters until the trained neural network provides an accurate output (in the above example, until the neural network's output is accurate with respect to the corresponding guidance for acquiring a medical image of the anatomical region using the medical imaging system). Training is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

The process of training the neural network(s) mentioned above therefore includes adjusting its parameters. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean squared error, or the Huber loss, or the cross entropy loss. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers" These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

One or more additional operations may also be performed in the computer-implemented method described above with reference to Fig. 1, as described in the examples below.

In one example, the method described with reference to Fig. 1 includes:
- receiving marker requirement information 180 representing one or more requirements of the anatomical orientation marker 150, 150'; and
- wherein the guidance 160 is generated based further on the one or more requirements of the anatomical orientation marker 150, 150'.

In this example, the marker requirement information may be received from a computer-readable storage medium. The marker requirement information may be received from a radiology information system "RIS", for example. In a related example, the one or more requirements include one or more of:
- a need to include the anatomical orientation marker 150, 150' in the medical image 170;
- an expected position of the anatomical orientation marker 150, 150' with respect to the anatomical region 120; and
- an expected type of the anatomical orientation marker 150, 150'.

Generating the guidance based on the one or more requirements of the anatomical orientation marker enables the guidance to be tailored to an anatomical orientation marker.

In this example, the need to include the anatomical orientation marker may be specified as a straightforward binary indication, i.e. yes or no. The need to include the anatomical orientation marker may be used to provide guidance such as an indication of a recommendation to include an anatomical orientation marker 150, 150' in the medical image 170, or an indication of a recommendation not to include an anatomical orientation marker 150, 150' in the medical image 170. In this example, the expected position of the anatomical orientation marker 150, 150' with respect to the anatomical region 120 may be specified as a boundary within which the anatomical orientation marker is expected to be located. The expected position of the anatomical orientation marker may be used to provide guidance such as an indication of a recommended position of the anatomical orientation marker 150, 150' with respect to the anatomical region 120, or an indication of a recommended position of an anatomical orientation marker 150, 150' in the medical image 170. Thus, it may be specified that an anatomical orientation marker is close to, but not overlapping with, the anatomical region. In this example, the expected type of the anatomical orientation marker may specify that a certain type of marker (e.g. left, versus right, AP versus PA, or that a marker that is specific to a medical facility, or a marker that includes an identification of the radiographer), should be present in the medical image.

In one example, the operation of analysing S120 the camera image data 110 further comprises determining, from the camera image data, a type and/or a current orientation of the anatomical region 120 within the field of view 130 of the medical imaging system 140. In this example, the guidance 160 is generated based on the type and/or the current orientation of the anatomical region 120.

Generating such guidance helps to avoid mistakes relating to the type and/or the current orientation of the anatomical region captured in a subsequently-acquired medical image. This may help to avoid the need to recall the subject to the imaging room in order to acquire a further medical image.

In this example, the type, and similarly the orientation, of the anatomical region may be determined using various image processing techniques. As described above, various object detection algorithms may be used for this purpose. For instance, in order to detect a type of the anatomical region, various objection detection algorithms that are based on convolutional neural network "CNN" architecture, or a You Only Look Once "YOLO" architecture, may be trained to using camera images that include various examples of anatomical regions. In order to determine a current orientation of the anatomical region, various known pose estimation algorithms may be used, including AlphaPose, OpenPifPaf, and OpenPose, for example. By way of an example, the output of the object detection algorithm may be to provide an indication that a foot captured in the camera image data is a left foot, and that an outer side of the foot is facing the X-ray source 140_{S}.

In a related example, the guidance 160 may be generated without registering a further image (e.g. a previously-acquired X-ray image, or an anatomical atlas image) to the anatomical region. The registration of such further images can be complex and result in a delay when the subject moves. It may also introduce errors to the analysis, e.g. the registration might inadvertently reverse the atlas with respect to the anatomical region. Thus, generating the guidance without registering a further image to the anatomical region obviates such drawbacks.

In another related example, the operation of analysing S120 the camera image data 110 comprises determining a current orientation of the anatomical region 120 within the field of view 130 of the medical imaging system 140. In this example, the operation of outputting S130 guidance comprises outputting, based on a comparison between the current orientation of the anatomical region 120 and a reference orientation of the anatomical region 120, an indication of a suitability of the current orientation and/ or an indication of one or more adjustments to the current orientation for obtaining the reference orientation.

Generating such guidance helps to avoid mistakes relating to the current orientation of the anatomical region captured in a subsequently-acquired medical image. This may help to avoid the need to recall the subject to the imaging room in order to acquire a further medical image.

By way of an example, if current orientation of a chest within a field of view of a medical imaging system is determined as PA, whereas the reference orientation is AP, the guidance may indicate that the chest currently has an incorrect orientation and/or that the subject should be rotated by 180 degrees in order to provide the correct orientation.

In a related example, the method comprises receiving orientation requirement information 190 representing the reference orientation of the anatomical region 110.

The orientation requirement information enables the guidance to be tailored to a specific reference orientation. In this example, the orientation requirement information 190 may be received from a computer-readable storage medium. The orientation requirement information 190 may be received from a radiology information system "RIS", for example.

In another example, a computer program product, is provided. The computer program product comprises instructions which when executed by one or more processors, cause the one or more processors to carry out a method of providing guidance for acquiring a medical image. The method comprises:
- receiving S110 camera image data 110 representing an anatomical region 120 within a field of view 130 of a medical imaging system 140;
- analysing S120 the camera image data 110 to determine a presence, or an absence, of an anatomical orientation marker 150, 150' within the field of view 130 of the medical imaging system 140; and
- outputting S130 guidance 160 for acquiring a medical image 170 of the anatomical region 120 using the medical imaging system 140, wherein the guidance 160 is generated based on the presence, or the absence, of the anatomical orientation marker 150, 150'.

In another example, a system 200 for providing guidance for acquiring a medical image. The system includes one or more processors 230 configured to:
- receive S110 camera image data 110 representing an anatomical region 120 within a field of view 130 of a medical imaging system 140;
- analyse S120 the camera image data 110 to determine a presence, or an absence, of an anatomical orientation marker 150, 150' within the field of view 130 of the medical imaging system 140; and
- output S130 guidance 160 for acquiring a medical image 170 of the anatomical region 120 using the medical imaging system 140, wherein the guidance 160 is generated based on the presence, or the absence, of the anatomical orientation marker 150, 150'.

It is noted that in addition to the one or more processors 230, the system 200 may also include one or more of: a medical imaging system for acquiring the medical image 170 (such as for example the X-ray imaging system 140 illustrated in Fig. 2); a display (such as the display 250 that is coupled to the X-ray imaging source 140s) for displaying the guidance, other outputs generated by the one or more processors 230, and so forth; a patient bed 240; and a user input device (not illustrated in Fig. 1) configured to receive user input in relation to the operations performed by the one or more processors 230, such as a keyboard, a mouse, a touchscreen, and so forth.

The above examples are to be understood as illustrative of the present disclosure, and not restrictive. Further examples are also contemplated. For instance, the examples described in relation to a computer-implemented method, may also be provided by the computer program product, or by the computer-readable storage medium, or by the system 200, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of providing guidance for acquiring a medical image, the method comprising:
- receiving (S110) camera image data (110) representing an anatomical region (120) within a field of view (130) of a medical imaging system (140);
- analysing (S120) the camera image data (110) to determine a presence, or an absence, of an anatomical orientation marker (150, 150') within the field of view (130) of the medical imaging system (140); and
- outputting (S130) guidance (160) for acquiring a medical image (170) of the anatomical region (120) using the medical imaging system (140), wherein the guidance (160) is generated based on the presence, or the absence, of the anatomical orientation marker (150, 150').

2. The computer-implemented method according to claim 1, wherein the method further comprises:
- receiving marker requirement information (180) representing one or more requirements of the anatomical orientation marker (150, 150'); and
- wherein the guidance (160) is generated based further on the one or more requirements of the anatomical orientation marker (150, 150').

3. The computer-implemented method according to claim 1, wherein the one or more requirements include one or more of:
- a need to include the anatomical orientation marker (150, 150') in the medical image (170);
- an expected position of the anatomical orientation marker (150, 150') with respect to the anatomical region (120); and
- an expected type of the anatomical orientation marker (150, 150').

4. The computer-implemented method according to any one of claims 1-3, wherein the guidance (160) is generated based on the presence of the anatomical orientation marker (150, 150'); and wherein the outputting (S130) guidance comprises one or more of:
- outputting an indication of a presence of the anatomical orientation marker (150, 150');
- outputting an indication of a suitability of a position of the anatomical orientation marker (150, 150') with respect to the anatomical region (120);
- outputting an indication of a recommended position of the anatomical orientation marker (150, 150') with respect to the anatomical region (120); and
- outputting an indication of a suitability of a type of the anatomical orientation marker (150, 150') for the anatomical region (120).

5. The computer-implemented method according to any one of claims 1 - 4, wherein the analysing (S120) the camera image data (110) further comprises determining, from the camera image data, a type and/or a current orientation of the anatomical region (120) within the field of view (130) of the medical imaging system (140); and
wherein the guidance (160) is generated based on the type and/or the current orientation of the anatomical region (120).

6. The computer-implemented method according to claim 5, wherein the analysing (S120) the camera image data (110) comprises determining a current orientation of the anatomical region (120) within the field of view (130) of the medical imaging system (140); and
wherein the outputting (S130) guidance further comprises outputting, based on a comparison between the current orientation of the anatomical region (120) and a reference orientation of the anatomical region (120), an indication of a suitability of the current orientation and/ or an indication of one or more adjustments to the current orientation for obtaining the reference orientation.

7. The computer-implemented method according to claim 6, wherein the method further comprises receiving orientation requirement information (190) representing the reference orientation of the anatomical region (110).

8. The computer-implemented method according to any one of claims 1-3, wherein the guidance (160) is generated based on the absence of the anatomical orientation marker (150, 150'), and wherein the outputting (S130) guidance comprises one or more of:
- outputting an indication of an absence of the anatomical orientation marker (150, 150');
- outputting an indication of a recommendation to include an anatomical orientation marker (150, 150') in the medical image (170);
- outputting an indication of a recommendation not to include an anatomical orientation marker (150, 150') in the medical image (170);
- outputting an indication of a recommended position of an anatomical orientation marker (150, 150') in the medical image (170); and
- outputting a prompt requesting user confirmation to include a virtual anatomical orientation marker in a proposed position in the medical image.

9. The computer-implemented method according to any previous claim, wherein the analysing (S120) the camera image data (110) comprises detecting the anatomical orientation marker (150, 150') and/or an orientation of the anatomical region (120), using an object detection algorithm.

10. The computer-implemented method according to any previous claim, wherein the analysing (S120) the camera image data (110) comprises inputting the camera image data (110) into a machine learning algorithm and generating the guidance (160) using the machine learning algorithm in response to the inputting;
wherein the machine learning algorithm is trained to generate the guidance (160) using training data comprising a plurality of instances of camera image data, each instance of camera image data representing an anatomical region within a field of view of a medical imaging system; and wherein the training data further comprises for each instance of camera image data, corresponding guidance for acquiring a medical image of the anatomical region using the medical imaging system, the corresponding guidance being determined based on the presence, or the absence, of an anatomical orientation marker within the field of view of the medical imaging system.

11. The computer-implemented method according to any previous claim, wherein the medical image (170) is an X-ray image.

12. The computer-implemented method according to any previous claim, wherein the camera image data (110) is acquired by a camera (210) having a field of view (220) overlapping the field of view (130) of the medical imaging system (140).

13. The computer-implemented method according to any previous claim, wherein the anatomical orientation marker (150, 150') is an X-ray anatomical side marker.

14. A computer program product comprising instructions which when executed by one or more processors (230), cause the one or more processors to carry out the method according to any one of claims 1-13.

15. A system (200) for providing guidance for acquiring a medical image, the system comprising one or more processors (230) configured to:
- receive (S110) camera image data (110) representing an anatomical region (120) within a field of view (130) of a medical imaging system (140);
- analyse (S120) the camera image data (110) to determine a presence, or an absence, of an anatomical orientation marker (150, 150') within the field of view (130) of the medical imaging system (140); and
- output (S130) guidance (160) for acquiring a medical image (170) of the anatomical region (120) using the medical imaging system (140), wherein the guidance (160) is generated based on the presence, or the absence, of the anatomical orientation marker (150, 150').
